# EUROPEAN PATENT APPLICATION

(11) **EP 4 174 753 A1**
(43) Date of publication of application: **03.05.2023**
(21) Application number: 21848899.7
(22) Date of filing: 08.06.2021
(51) Int. Cl.: G06Q 50/10, G06Q 50/22, G16H 10/60, G06Q 30/06, A61B 5/145, A61B 5/00

(54) **METHOD FOR PROVIDING GUIDANCE ON USE PERIOD OF SENSOR**

(30) Priority: 29.07.2020 KR 20200094538
(71) Applicant: i-Sens, Inc., Seoul 06646 (KR)
(72) Inventor: KANG, Bo Weol, Seoul 06646 (KR); LEE, Jin Won, Seoul 06646 (KR)
(74) Representative: BCKIP Part mbB
(86) International application number: PCT/KR2021/007135
(87) International publication number: WO 2022/025411

(57) **Abstract**

The present disclosure relates to a method for providing guidance on a use period of a sensor and, more specifically, to a method for providing guidance on a use period of a sensor, which accurately provides guidance to a user about a use period of a sensor by calculating the number of sensors possessed by the user and the total use period from the use period of each sensor so as to determine whether the remaining use period has arrived at an order guidance period or to determine whether the remaining use period of the last sensor has arrived at the order guidance period, on the basis of a sensor usage history of the user, and enable the user to order sensors on the basis of the guidance.

## Description

### TECHNICAL FIELD

The present disclosure relates to a method for guiding a usage period of a sensor, and, more specifically, a method for guiding a usage period of a sensor by calculating a total usage period from the number of sensors possessed by a user and a usage period for each sensor and determining whether the remaining usage period of the sensors has reached an order guidance period or determining whether a remaining usage period of a last sensor has reached an order guidance period, thereby accurately guiding the user regarding the usage period of the sensors and allowing the user to order a sensor based on this.

### BACKGROUND

Diabetes is a major cause of death and a cause of disability worldwide, and therefore, many people have health problems due to diabetes. Specially, diabetes is a serious disease that causes heart and kidney disease, blindness, nerve damage and high blood pressure. According to a long-term clinical study, the incidence of complications can be significantly reduced by appropriately managing blood glucose levels. Therefore, it is important to continuously manage diabetes, an important factor is self-monitoring of blood glucose levels.

In response to this demand, a self-diagnosis biometer in which a user can check a blood glucose level of the user by himself or herself has been widely distributed and used. A conventional blood glucose meter measures the blood glucose level of the user by putting the user's blood on a sensor strip, which is a test strip. Accordingly, the sensor strip with the blood is inserted into the blood glucose meter, and the blood glucose level measured through the sensor strip is displayed on the blood glucose meter.

At this time, the blood glucose meter receives an electrical signal generated by an electrochemical reaction between the collected blood and the reactant in the sensor strip, and measures the blood glucose level. Such a blood-collect-type blood glucose meter (finger prick method) helps diabetic patients to manage blood glucose, but it is difficult to accurately identify the blood glucose levels which are being frequently changed because it shows only the result at the time of the measurement.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

To overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a body attachable unit configured to be insertable into a human body to measure blood glucose by collecting a body fluid such as blood of a user, and a communication terminal for communicating with the body attachable unit and displaying a blood glucose level measured by the body attachable unit.

The body attachable unit includes a sensor that is partially inserted into the body and generates a biosignal representing the user's blood glucose value from the body fluid, and a transmitter that processes the biosignal received from the sensor, measures the blood glucose value of the user, and transmits blood glucose information including the measured blood glucose value to a communication terminal. Hereinafter, various types of sensors continuously used for a certain use period, including a body attachable unit, will be referred to as a sensor for convenience of description.

The sensor for continuous blood glucose measurement cannot be used permanently and has a lifespan of a certain period, so when the usage period expires, it needs to be replaced.

When the use period ends, the sensor for continuous blood glucose measurement no longer measures the blood glucose of the body from a time point of the end of the use period, and data transmission to the communication terminal is no longer performed. In some cases, the power of the continuous blood glucose measurement sensor may be automatically turned off.

When the usage period of the sensor for continuous glucose measurement is over, the user cannot receive blood glucose information from the sensor for continuous glucose measurement anymore, so the sensor for continuous glucose measurement sensor needs to be replaced, and a technique for notifying to the user that the use period of the sensor for continuous glucose measurement sensor being currently used is about to end or informing the user of the remaining use period of the sensor for continuous blood glucose measurement being currently in use. Based on this, the user may order a new sensor for continuous blood glucose measurement, replace the sensor for continuous blood glucose measurement sensor with a new sensor continuous blood glucose measurement after the usage period of the sensor for continuous blood glucose measurement ends, and continuously measure blood glucose.

However, the frequent notification of the remaining usage period or the end of the usage period of the sensor for continuous blood glucose measurement causes inconvenience to the user, and furthermore, when the user has a plurality of sensors for continuous blood glucose measurement, the notification of the remaining usage period or the end of the use period of individual continuous blood glucose measurement sensors is unnecessary and gives only inconvenience to the user.

### SUMMARY

### Technical Problem

The present disclosure is invented to solve problems in conventional technique of notifying a remaining usage period of a sensor, and the purpose of the present disclosure is for providing a method of guiding a use period of a sensor to a user by determining one or more sensors possessed by the user and determining whether a remaining usage period of the sensors possessed by the user has reached an order guidance period.

Another purpose of the present disclosure is for providing a method of guiding a use period of a sensor to a user by calculating a total usage period from the number of sensors possessed by the user and a usage period for each sensor, and determining whether a remaining usage period of the sensors possessed by the user has reached an order guidance period based on the total usage period.

Another purpose of the present disclosure is for providing a method of guiding a use period of a sensor to a user by, when it is determined that the user uses a last sensor based on a usage history of sensors possessed by the user, determining whether a remaining usage period of the last sensor has reached an order guidance period.

Another purpose of the present disclosure is for providing a method of guiding a use period of a sensor to a user by, when the user adds a new sensor or when a sensor currently in use is removed with the remaining usage period due to a defect or usage error, updating sensor usage information of sensors possessed by the user, re-calculating the remaining usage period, and informing the user of a usage period of the sensors based on the re-calculated usage period.

Another purpose of the present disclosure is for providing a method of guiding a use period of a sensor to a user by, when multiple sensors are packaged in one box and shipped or sold, simultaneously recognizing and registering the packaged multiple sensors as possessed sensors using a sensor identifier placed on or in the box, and, when one or some of the registered multiple sensor are transferred to another person according to the user's selection, guiding the usage period of the sensors to the user by excluding the transferred sensor(s).

### Solution to Problem

To accomplish the purpose of the present disclosure, a method of guiding a use period of a sensor according to an embodiment of the present disclosure may comprise: determining one or more sensors possessed by a user; determining whether a remaining usage period of the one or more sensors has reached an order guidance period; and when the remaining usage period of the one or more sensors has reached the order guidance period, providing an order guidance message to the user, wherein the one or more sensors are continuously usable for a set use period.

Preferably, according to an embodiment of the present disclosure, the determining of whether the remaining usage period of the one or more sensors possessed by the user has reached the order guidance period may comprise: calculating a total usage period of the one or more sensors possessed by the user based on a usage period of each sensor possessed by the user and a number of the one or more sensors possessed by the user; and determining whether the remaining usage period of the one or more sensors possessed by the user has reached the order guidance period based on the total usage period.

Preferably, according to an embodiment of the present disclosure, the determining of whether the remaining usage period of the one or more sensors has reached the order guidance period may further comprises determining whether one of the one or more sensors possessed by the user is used, wherein the total usage period of the one or more sensors possessed by the user may be calculated based on the usage period of each sensor possessed by the user and the number of the one or more sensors possessed by the user based on a time point when the user uses the one of the one or more sensors.

Preferably, according to an embodiment of the present disclosure, the method of guiding the use period of the sensor may further comprise: additionally register a new sensor during use of the one or more sensors; when the new sensor is additionally registered, changing information of the one or more sensors possessed by the user; and re-calculating the total usage period of the one or more sensors possessed by the user based on the usage period of each sensor possessed by the user and the number of the one or more sensors possessed by the user, wherein the determining of whether the remaining usage period of the one or more sensors has reached the order guidance period may be performed based on the re-calculated total usage period of the one or more sensors.

Preferably, according to an embodiment of the present disclosure may further comprise: determining whether a sensor registered as a sensor currently being used by the user is removed; at the time of removing the sensor registered as the sensor currently being used by the user, determining whether a remaining usage period of the sensor registered as the sensor currently being used by the user exists; and when the remaining usage period of the sensor currently being used exists, re-calculating the total usage period of the one or more sensors by subtracting the remaining usage period of the sensor currently being used from the total usage period of the one or more sensors possessed by the user, whether the remaining usage period of the one or more sensors has reached the order guidance period may be determined based on the re-calculated total usage period of the one or more sensors.

Preferably, the determining of whether the sensor registered as the sensor currently being used by the user is removed may comprise, when a sensor different from the sensor registered as the sensor currently being used is newly registered to be used, determining that the sensor registered as the sensor currently being used is removed.

Preferably, according to another embodiment of the present disclosure, the determining of whether the remaining usage period of the one or more sensors has reached the order guidance period may comprise: determining whether a last sensor is being currently used among the one or more sensors possessed by the user based on use history of the one or more sensors possessed by the user; when it is determined that the last sensor is being currently used among the one or more sensors possessed by the user, determining a remaining usage period of the last sensor; and determining whether the remaining usage period of the last sensor has reached the order guidance period.

Preferably, according to another embodiment of the present disclosure, the method of guiding the use period of the sensor may further comprise: additionally registering a new sensor during use of the one or more sensors; and when the new sensor is additionally registered, changing information of the one or more sensors possessed by the user, wherein whether the last sensor is being currently used among the one or more sensors possessed by the user may be determined based on the use history of the one or more sensors possessed by the user.

Here, the one or more sensors may be one or more sensors for continuous measurement of blood glucose.

Preferably, according to an embodiment of the present disclosure, in the method of guiding the use period of the sensor, one or more sensors may be provided to be packaged in a box, a code corresponding to each of the packaged one or more sensors may be disposed on or in the box, a sensor identifier of each of the one or more sensors may be acquired from the code, and the packaged one or more sensors may be registered as the one or more sensors possessed by the user based on the acquired sensor identifier.

Preferably, according to another embodiment of the present disclosure, in the method of guiding the use period of the sensor, a code having a sensor identifier may be disposed on each of one or more sensors, the sensor identifier may be acquired from the code, the one or more sensors may be registered as the one or more sensors possessed by the user based on the acquired sensor identifier.

According to an embodiment of the present disclosure, the method of guiding the use period of the sensor may further comprise: providing a list of the one or more sensors possessed by the user to the user in response to a request of the user; and when a deletion request for selecting a sensor which the user requests to delete from the list is input, updating the list by deleting the selected sensor from the list, whether the remaining usage period of the one or more sensors possessed by the user has reached the order guidance period may be determined based on the updated list.

### Advantageous Effects of Invention

A method for guiding a usage period of a sensor according to embodiments of the present disclosure has various effects as follows.

First, according to an embodiment of the present disclosure, a method of guiding a use period of a sensor to a user can accurately guide the usage period of the sensor possessed by the user so that the user can order the sensor based on this by calculating a total usage period from the number of sensors possessed by the user and an usage period for each sensor, and determining whether a remaining usage period of the sensors possessed by the user has reached an order guidance period based on the total usage period.

Second, according to an embodiment of the present disclosure, a method of guiding a use period of a sensor to a user can accurately guide the usage period of the sensor possessed by the user so that the user can order the sensor based on this by, when it is determined that the user uses a last sensor based on a usage history of sensors possessed by the user, determining whether a remaining usage period of the last sensor has reached an order guidance period.

Third, according to an embodiment of the present disclosure, a method of guiding a use period of a sensor to a user can accurately guide the usage period of the sensor possessed by the user by, when the user adds a new sensor or when a sensor currently in use is removed with the remaining usage period due to a defect or usage error, updating sensor usage information of sensors possessed by the user, re-calculating the remaining usage period based on the updated sensor usage period, and accurately informing the user of a usage period of the sensors based on the re-calculated usage period.

Fourth, according to an embodiment of the present disclosure, a method of guiding a use period of a sensor to a user can easily register one or more sensors possessed by the user by, when multiple sensors are packaged in one box and shipped or sold, simultaneously recognizing and registering the packaged multiple sensors as possessed sensors using a code placed on or in the box.

Fifth, according to an embodiment of the present disclosure, a method of guiding a use period of a sensor to a user can accurately guide the usage period of the sensor possessed by the user by, when one or some of the registered multiple sensor are transferred to another person according to the user's selection, guiding the usage period of the sensors to the user by excluding the transferred sensor(s).

### BRIEF DESCRIPTION OF THE DRAWINGS

FIG. 1 is a diagram for explaining a system for use of a sensor for continuous blood glucose measurement according to an embodiment of the present disclosure.
FIG. 2 is a diagram for explaining a packaging state of a sensor.
FIG. 3 is a functional block diagram for explaining a user terminal for guiding a usage period of a sensor to a user according to an embodiment of the present disclosure.
FIG. 4 is a functional block diagram for explaining an example of a registration management unit according to an embodiment of the present disclosure.
FIG. 5 is a functional block diagram for explaining an order guidance determination unit according to an exemplary embodiment of the present disclosure.
FIG. 6 is a functional block diagram for explaining an order guidance determination unit according to another embodiment of the present disclosure.
FIG. 7 is a flow chart for explaining a method for guiding a usage period of a sensor according to an embodiment of the present disclosure.
FIG. 8 is a flow chart for explaining a method for determining whether an order guidance period has arrived according to an embodiment of the present disclosure.
FIG. 9 is a flow chart for explaining a method for determining whether an order guidance period has arrived according to another embodiment of the present disclosure.
FIG. 10 illustrates an interface screen provided in relation to a usage period of sensors possessed by a user according to an embodiment of the present disclosure.
FIG. 11 is a flowchart for explaining a method of registering a sensor as a sensor possessed by a user according to an embodiment of the present disclosure.
FIG. 12 illustrates an interface screen for updating a sensor list according to an embodiment of the present disclosure.

### DETAILED DESCRIPTION OF EMBODIMENTS

The technical terms used in the present disclosure are only for the purpose of describing exemplary embodiments, and they are not intended to limit the present invention. Also, unless otherwise defined, all technical terms used herein should be construed as having the same meaning as commonly understood by those skilled in the art, and should not be interpreted as being excessively inclusive or excessively restrictive. In addition, when a technical term used herein is an erroneous technical term that does not accurately represent the idea of the present invention, it should be understood as replacing the term by a technical term which can be properly understood by those skilled in the art.

Further, singular expressions used in the present specification include plural expressions unless they have definitely opposite meanings. In the present application, it shall not be construed that terms, such as "including" or "comprising", various constituent elements or steps described in the specification need to be all essentially included, and it shall be construed that some constituent elements or steps among the various constituent elements or steps may be omitted, or additional constituent elements or steps may be further included.

Also, it should be noted that the accompanying drawings are merely illustrated to easily explain the spirit of the invention, and therefore, they should not be construed to limit the spirit of the invention by the accompanying drawings.

Hereinafter, a method for guiding a usage period of a sensor according to the present disclosure will be described in more detail with reference to the accompanying drawings.

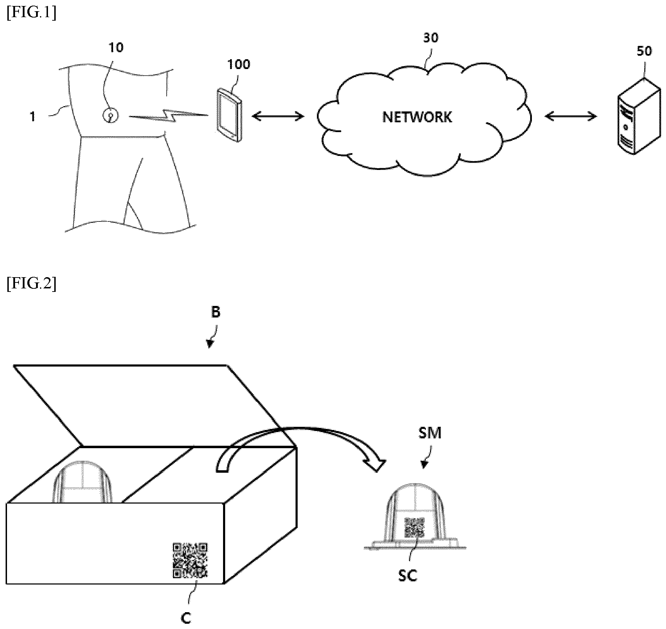

FIG. 1 is a diagram for explaining a system for use of a sensor for continuous blood glucose measurement according to an embodiment of the present disclosure.

Looking in more detail with reference to FIG. 1, a user terminal (100) and a management server (50) are connected to a network (30). Here, various types of wired or wireless networks capable of transmitting and receiving data between the user terminal (100) and the management server (50) may be used as the network (30).

Meanwhile, the user terminal (100) is connected to the sensor (10) for communication to receive biometric information such as blood glucose information from the sensor (10). Preferably, the user terminal (100) and the sensor (10) may communicate with each other through short-range communication, Bluetooth, infrared communication, or the like. Here, the sensor (10) is described as a sensor for continuous blood glucose measurement, but the sensor (10) includes various types of sensors that are continuously used for a certain period of time depending on the field to which the present disclosure is applied.

The user terminal (100) stores the biometric information received from the sensor (10) for a certain period of time, and can transmis the stored biometric information to the management server (50) in real time or at a time point when the user terminal (100) can communicate with the management server (50).

The management server (50) receives sensor information of a sensor possessed or owned by the user from the user terminal (100), or receives sensor information of a sensor currently being used by the user, or maps biometric information received from the user terminal and stores and manages it, or, when there is a request of the user terminal (100), provides the biometric information of the user to the user terminal (100) or can provide various types of reports or advice related to the user's biometric information.

The user terminal (100) acquires an identifier of the sensor (10), and registers a sensor currently being used by the user with the acquired identifier of the sensor (10). In particular, when the user terminal (100) and the sensor (10) communicate each other through Bluetooth, the user terminal (100) receives a sensor identifier from the sensor (10) and registers the received sensor identifier the sensor (10) as a sensor to be currently used by the user.

In the present disclosure, a plurality of sensors may be packaged together in one box and shipped or sold, and as shown in FIG. 2, two sensors may be packaged together in one box (B) and shipped or sold. A comprehensive code (C) capable of recognizing all identifiers of a plurality of packaged sensors (SM) is disposed on or in the box (B), and each sensor (SM) has an individual code (SC) capable of recognizing an identifier of an individual sensor. Here, only sensor may be packed in the box (B), or an applicator for inserting the sensor may be packed together with the sensor.

The user can obtain identifiers of all sensors packaged in the box (B) simultaneously using the comprehensive code (C) disposed on or in the box (B) through the user terminal (100), or obtains an identifier of each sensor through an individual code (SC) disposed on or in each sensor (SM). The acquired sensor identifier is registered as a sensor possessed or owned by the user, and the user terminal (100) can determine a remaining usage period of the sensor possessed or owned by the user based on the number of sensors possessed or owned and the period of use for each sensor. In addition, the user terminal (100) determines whether an order guidance period has arrived based on the remaining usage period of the sensor possessed or owned by the user, and, when the order guidance period has arrived, informs that the usage period of the sensor possessed or owned by the user has expired, or orders a new sensor.

Here, the usage period for each sensor may be determined based on the sensor identifier.

Here, the comprehensive code (C) or individual code (SC) is a code that can be visually read, such as a barcode or QR code, and a sensor identifier can be obtained by reading a comprehensive code (C) placed on or in the box (B) through a code reading means provided in the user terminal 100 or a sensor identifier can be obtained by reading an individual code (SC) placed on the sensor (SM). Depending on the field to which the present disclosure is applied, the comprehensive code (C) or individual code (SC) may be an RFID chip, and the sensor identifier is obtained from the comprehensive code (C) disposed on or in the box (B) through the RFID reading means provided in the user terminal, or the sensor identifier can be obtained from the individual code (SC) disposed on or in the sensor (SM).

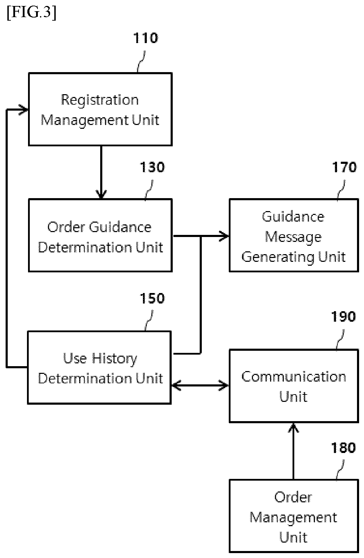

FIG. 3 is a functional block diagram for explaining a user terminal for guiding a usage period of a sensor to a user according to an embodiment of the present disclosure.

Referring to FIG. 3 in more detail, a registration management unit (110) registers a sensor possessed or owned by a user by acquiring a sensor identifier. The registration management unit (110) includes a code acquiring means capable of reading or receiving a code, and the registration management unit (110) determines whether the sensor identifier is valid from the read or received code, and, if the sensor identifier is valid, registers a sensor corresponding to the sensor identifier as a senser owned by the user in a sensor list. Preferably, the registration management unit (100), when acquiring a new sensor identifier while using a sensor, can additionally register a new sensor to the sensor list, or according to selection of the user, can delete one or some sensors from the sensor list among the sensors possessed by the user.

A usage history management unit (150) provides usage history information of a sensor to the registration management unit (110) by determining whether a sensor possessed by a user is used, and the registration management unit (110) updates or renews sensor information of sensors owned by the user in the sensor list based on the usage history information. That is, the registration management unit (110) sorts into and separately manages one or more sensor completely used by the user, one or more sensors being currently in use by the user, and one or more sensors to be used later among the sensors owned by the user in the sensor list.

Preferably, when being connected to a sensor through a communication unit (190) and receiving a sensor identifier from the sensor, the usage history management unit (150) may determine that the sensor of the received sensor identifier is being currently used. In addition, if the sensor identifier received from the sensor connected through the communication unit (190) is different from a sensor identifier of a sensor registered as a used sensor, the use history determination unit (150) removes the previously registered used sensor and determines that a new sensor is registered as a currently used sensor.

An order guidance determination unit (130) calculates the remaining period of use of the sensor possessed by the user based on the number of sensors owned by the user and the period of use of each sensor based on the sensor information of the sensor list, and determines whether the remaining period of use has reached an order guidance period. When the remaining usage period reaches the order guidance period, the order guidance determining unit (130) guides the user through the guidance message generating unit (170) that the usage period of the sensor is over, or guides to order a new sensor.

When a user command for requesting an order of a new sensor is received through the user terminal, an order management unit (180) requests the order for the new sensor by transmitting order information of the user input through the user terminal or pre-stored in the user terminal through the communication unit (190) to the management server. Preferably, the order management unit (180) activates an order interface screen into which the number of sensors to be ordered, orderer information, payment information, etc. can be input, and the user can easily order sensors through the order interface screen.

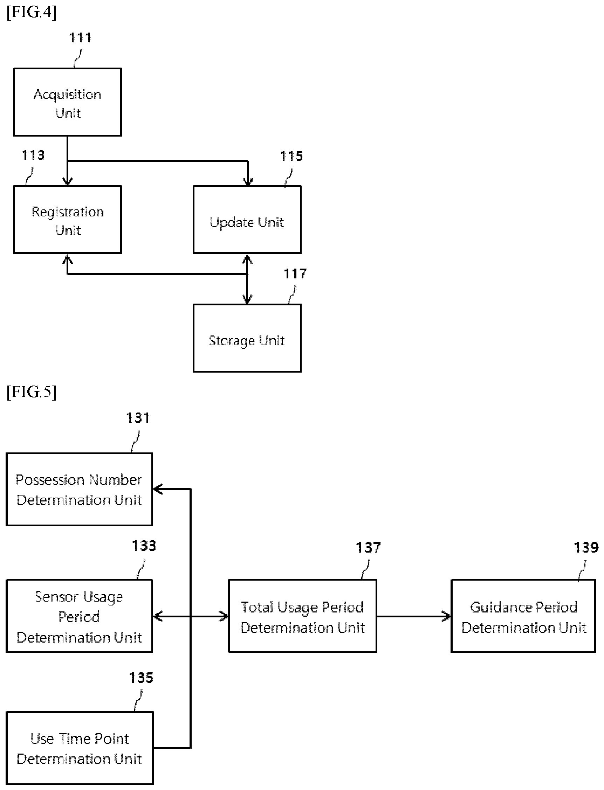

FIG. 4 is a functional block diagram for explaining an example of a registration management unit according to an embodiment of the present disclosure.

Referring to FIG. 4 in more detail, an acquisition unit (111) obtains a sensor identifier from a comprehensive code disposed on or in a box or an individual code disposed on a sensor. Here, the acquisition unit (111) may use various means for reading or receiving a code according to the type of the disposed comprehensive code or individual code.

When a registration unit (113) acquires a sensor identifier from a comprehensive code or an individual code through the acquisition unit (111), the registration unit (113) registers a sensor corresponding to the acquired sensor identifier as a sensor possessed by the user to a storage unit (117). That is, the acquired sensor identifier at the sensor list stored at the storage unit (117) is added as a new sensor to register the sensor owned by the user.

Meanwhile, when an update unit (115) receives usage history information of the sensor from the usage history management unit, the update unit (115) updates sensor information about the usage state of the sensor registered in the sensor list. For example, when receiving a sensor identifier of a currently used sensor from the usage history management unit, a sensor corresponding to the received sensor identifier is updated as a used sensor, and when a sensor identifier which is different from the sensor identifier of the used sensor is received from the usage history management unit again, the previously used sensor is removed and sensor information about the sensor usage state is updated in the sensor list to update that the sensor of the received sensor identifier is newly used as a used sensor.

In this way, the registration unit (113) and the update unit (115) classify sensors possessed by the user in the sensor list into completely used sensors already completely used by the user, used sensors currently in use, and usable sensors that can be used in the future, and manage their registration.

FIG. 5 is a functional block diagram for explaining an order guidance determination unit according to an exemplary embodiment of the present disclosure.

Referring to FIG. 5 in more detail, a possession number determination unit (131) determines the number of sensors possessed by the user in the sensor list. The possession number determination unit (131) determines the number of sensors possessed by the user from sensors, which are in use or not in use, based on information on the state of usage of the sensors in the sensor list.

A sensor usage period determination unit (133) determines a usage period for each sensor possessed by the user based on a sensor identifier of a sensor possessed by the user.

A use time point determination unit (135) determines a use time point of the sensor possessed by the user. That is, the use time point determining unit (135) determines a time point when the user starts using the sensor possessed by the user. Here, the use time point determining unit (135) may determine that a sensor of a corresponding sensor identifier starts to be used from a time point when the use history determining unit receives the sensor identifier of the sensor currently being used by the user through the communication unit.

A total usage period determination unit (137) calculates the total usage period of the sensors possessed by the user based on the number of sensors possessed by the user, the period of use for each sensor, and the use time point of the sensor. When a new sensor is additionally registered, the total usage period determination unit (137) recalculates the total period of use based on the number of one or more additionally registered sensors and the period of use, and, when the currently used sensor is removed, the total usage period determination unit (137) recalculates the total usage period by determining whether there is a remaining period of use of the removed sensor(s).

On the other hand, it is preferable that the user has a plurality of sensors and continuously uses the sensors without interruption, but if the user uses the sensor again after stopping using that sensor for a certain period of time, the total usage period determination unit (137) re-determines the use time point of the sensor and recalculates the total usage period from the time point when the sensor is used.

A guidance period determination unit (139) determines whether an order guidance period has arrived by considering the total period of use of the sensors possessed by the user and time period elapsed after the calculation of the total usage period.

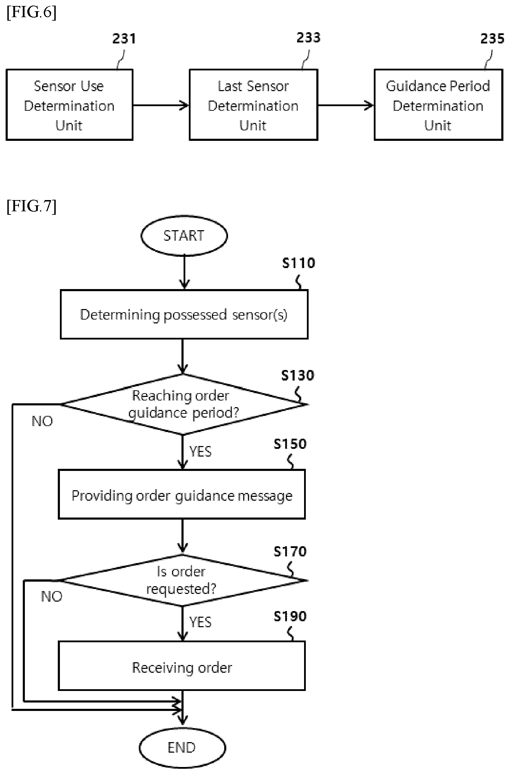

FIG. 6 is a functional block diagram for explaining an order guidance determination unit according to another embodiment of the present disclosure.

Referring to FIG. 6 in more detail, a sensor use determination unit (231) determines a sensor currently being used by the user among the sensors possessed by the user.

A last sensor determination unit (233) determines whether a sensor currently being used by the user is a last sensor among the sensors possessed by the user based on use state information of the sensors possessed by the user. Here, the number of last sensors can be preset by the user, and when the number of last sensors is set to n (n is a natural number), if the number n of sensors remains, it is determined as the last sensor. For example, if n is set to 2, if there are 2 sensors currently available to the user based on the use status information of the sensors possessed by the user, it can be determined as the last sensor, and if n is set to 1, if there is 1 sensor currently available to the user based on the use status information of the sensors possessed by the user, it can be determined as the last sensor. In this way, by setting the number of the last sensor(s) according to the user's selection, alarm time for the last sensor according to the user's tendency or preference can be adjusted.

When the sensor currently used by the user is a last sensor, a guidance period determination unit (235) determines a remaining usage period of the last sensor, and determines whether the remaining usage period of the last sensor has reached a set order guidance period.

FIG. 7 is a flow chart for explaining a method for guiding a usage period of a sensor according to an embodiment of the present disclosure.

Referring to FIG. 7 in more detail, information on sensors possessed by the user is determined (S110). For example, sensor information such as the number of sensors currently possessed by the user, the period of use for each sensor, the state of use of the sensors, and the use time point of the sensors in the sensor list is determined.

The remaining usage period of the sensors possessed by the user is calculated based on the determined sensor information, and whether the remaining usage period of the sensors possessed by the user has reached an order guide period (S 130).

When the remaining usage period of the sensors possessed by the user is compared with the order guidance period and the remaining usage period of the sensors possessed by the user reaches the order guide period, an order guidance message is output to the user terminal to inform the user (S 150). Preferably, the order guidance message as well as information on the remaining usage period of the sensors possessed by the user may be provided to the user simultaneously.

Whether a user command for requesting an order for a new sensor is input through the user terminal is determined (S 170), and when the user command for requesting the order for the new sensor is input, an ordering interface is activated in the user terminal and a sensor is ordered using order information input through the ordering interface or order information pre-stored in the user terminal (S190).

Here, the order information input through the ordering interface includes the type of a sensor to be ordered by the user, the number of sensors, the orderer's name, address, requests for ordering, payment information, and so on.

Here, the pre-stored order information is information which was previously input and stored by the user through the ordering interface when a user placed a previous order, and may be, for example, the type of a sensor to be ordered by the user, the number of sensors, the orderer's name, address, requests for ordering, payment information, and so on.

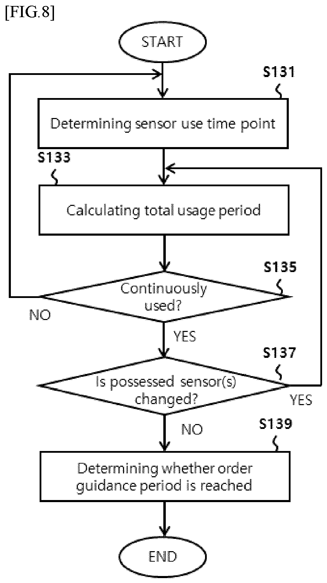

FIG. 8 is a flow chart for explaining a method for determining whether an order guidance period has arrived according to an embodiment of the present disclosure.

Referring to FIG. 8 in more detail, a time point when a user starts to use a sensor among the sensors possessed by the user is determined (S131). As an example of determining a time point when the user starts to use the sensor, a time point when a user terminal and a sensor communicate with each other and the user terminal receives a sensor identifier from the sensor is determined as the time point when the sensor starts to be used. As another example of determining when the user starts to use the sensor, a time point when a user inputs or selects a sensor identifier of a sensor to be used from the sensor list output to the user terminal may be determined as a time point when a corresponding sensor starts to be used.

A total usage period of the sensors possessed by the user is calculated based on sensor information of the sensors possessed by the user and the time point when sensors start to be used (S133). The total usage period of the sensors possessed by the user is calculated from the number of sensors possessed by the user, the usage period of each sensor, the state of use of the sensors, and the time of use of the sensors.

Whether after using one of the sensors possessed by the user another sensor is continuously used is determined (S135). Whether or not the user continuously uses another sensor can be determined by determining that when a sensor having a sensor identifier different from the sensor identifier of the sensor registered as a sensor currently used by the user is continuously registered as a use sensor, the sensor is continuously used.

When the user does not continuously use the sensor, a time point when a sensor having a sensor identifier different from a sensor identifier of a sensor registered as a previously used sensor is registered as a use sensor is determined as a sensor use time point, and a total usage time of sensors possessed by the user is recalculated from the sensor information of the sensors possessed by the user and the sensor use time point.

When the user continuously uses the sensor, whether sensor information of the sensors possessed by the user has been changed is determined (S137). For example, if a user additionally registers a new sensor, the total usage period including a usage period of the additionally registered sensor is recalculated, and if a use sensor is removed in a state that a remaining usage period remains, the total usage period is recalculated by deducting the remaining usage period from the total usage period.

Preferably, when a new sensor is additionally registered, the additionally registered sensor is additionally registered as a usable sensor in the sensor list, and when the currently used sensor is removed, the removed sensor is registered as a completely used sensor to the sensor list.

More preferably, when a use sensor is removed, the state information of use of the removed use sensor regarding whether the use sensor is removed after the use sensor is normally used during the usage period or whether the use sensor is abnormally removed with the remaining usage period left can be stored in the sensor list.

Whether a remaining usage period calculated by subtracting a period which has elapsed after a sensor use time point from the calculated total usage period has reached an order guidance period is determined (S139).

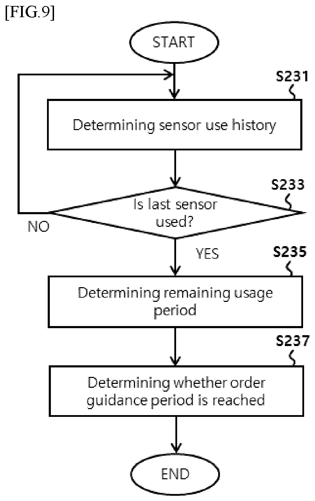

FIG. 9 is a flow chart for explaining a method for determining whether an order guidance period has arrived according to another embodiment of the present disclosure.

Referring to FIG. 9 in more detail, a sensor use history of sensors possessed by the user is determined (S231). That is, the sensors owned by the user are sorted into completely used sensors, currently used sensors, and usable sensors, and are registered in the sensor list, and the sensor use history of sensors owned by the user is determined in the sensor list.

Based on the sensor use history of the sensors possessed by the user, whether the sensor currently being used by the user is a last sensor among the possessed sensors is determined (S233).

If the sensor currently being used by the user is a last sensor, the remaining usage period of the last sensor is determined (S235). The remaining usage period of the last sensor can be calculated based on the number of sensor(s) set as last sensor(s), the usage period of the sensor(s) determined as the last sensor(s), and the remaining usage period of the currently used sensor. For example, if 2 sensors are set as the last sensors, the last sensors are the currently used sensor and 1 usable sensor, and the remaining usage period of the last sensors is calculated by adding the remaining usage period of the currently used sensor and the use period of one usable sensor.

By comparing the calculated remaining usage period of the last sensor(s) with an order guidance period, whether the remaining usage period of the last sensor(s) has reached the order guidance period is determined (S237).

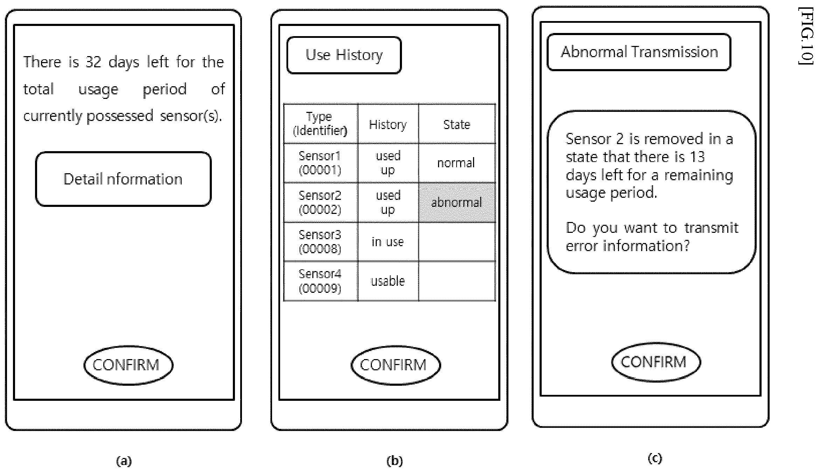

FIG. 10 illustrates an interface screen provided in relation to a usage period of sensors possessed by a user according to an embodiment of the present disclosure.

As shown in FIG. 10(a), information on the total usage period of the sensors possessed by the user is provided. When detailed information on sensors possessed by the user is requested, as illustrated in FIG. 10(b), sensor identifiers possessed by the user, usage history information of the sensors, and usage state information are output. In the sensor usage history information, information about whether the possessed sensors are used or not is indicated, and in the use state information, information about whether the used sensor is normally used or abnormally completed its use is indicated.

As shown in FIG. 10(c), when the use of a sensor is abnormally completed, the user may transmit abnormal completion information of the sensor of which use is abnormally completed to the management server. For example, the abnormal completion information such as whether the sensor of which use is completed is removed with a remaining usage period due to sensor failure or whether the sensor of which use is completed is removed with a remaining usage period due to a user's operation error may be transmitted to the management server.

The management server may perform an after-sales service such as sensor replacement in the case of sensor failure based on the abnormal completion information, and may guide the user once again a method of how to use the sensor in the case of an operation error.

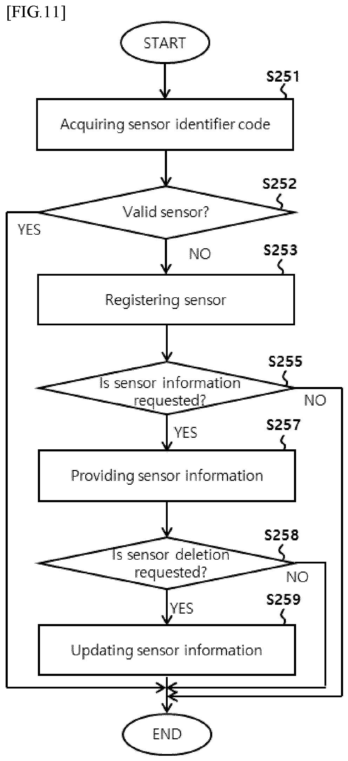

FIG. 11 is a flowchart for explaining a method of registering a sensor as a sensor possessed by a user according to an embodiment of the present disclosure.

Referring to FIG. 11 in more detail, a sensor identifier is acquired through a comprehensive code disposed on or in a box or an individual code disposed on a sensor (S251).

Whether the acquired sensor identifier is of a valid sensor is determined based on the acquired sensor identifier (S252). That is, whether the acquired sensor identifier is of a previously registered sensor is determined by comparing the acquired sensor identifier with a sensor identifier registered in the sensor list possessed by the user, or the validity of use of the sensor, such as whether the sensor past expiration date or whether the sensor is a defective sensor reported as a sensor to be replaced by the management server is determined based on the acquired sensor identifier, or the validity of use of the sensor, such as whether the sensor is a sensor which can be used in conjunction with an application running on the user terminal, is determined based on the acquired sensor identifier.

When the acquired sensor identifier is determined to be a valid sensor, the sensor of the acquired sensor identifier is registered as a sensor possessed by the user (S253).

Whether a user command for requesting sensor information of sensors possessed by the user is input through the user terminal is determined (S255), and if there is the request for the sensor information, the sensor information of sensors possessed by the user is provided and output in the form of a sensor list (S257).

The user may provide the possessed sensor to another user, and when a user command for selecting a specific sensor from the sensor list and requesting the removal of the selected sensor is input (S258), the sensor list is updated by deleting the sensor requested for the removal from the sensor list possessed by the user (S259). When the sensor information of sensors possessed by the user is updated by deleting the sensor from the sensor list, an order guidance message is provided by recalculating the remaining usage period of the sensors possessed by the user based on the updated sensor information.

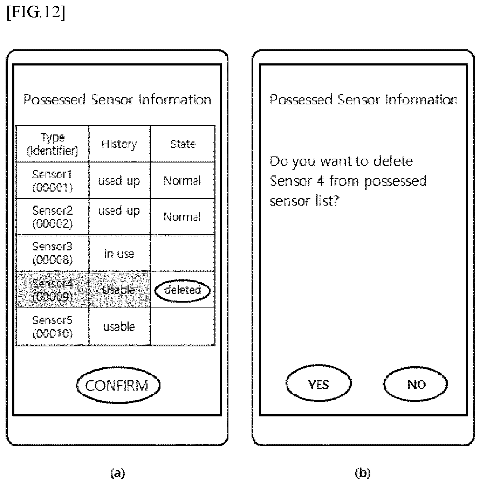

FIG. 12 illustrates an interface screen for updating a sensor list according to an embodiment of the present disclosure.

As shown in FIG. 12(a), when requesting sensor information on sensors possessed by the user, a sensor list of sensors possessed by the user is output. If the user wants to transfer sensor 4 among sensors registered as usable sensors to another user and the sensor 4 is selected by the user, an icon for deletion to ask if the sensor 4 is to be deleted is activated.

When the icon for deletion is selected as shown in FIG. 12(b), an interface screen for confirming whether to delete the sensor 4 from the sensor list possessed by the user is activated, and the sensor list possessed by the user is updated to delete the sensor 4 according to an end user confirmation command.

Meanwhile, the exemplary embodiments of the present disclosure described above can be implemented through programs executable at computers, and can be operated in a generalpurpose digital computer executing the programs using computer readable medium.

The above-referenced computer readable medium comprises storage medium such as magnetic storage media (e.g., ROM, floppy disks, hard disks, etc.), optical recording media (e.g., CD-ROMs, DVDs, etc.), and carrier waves (e.g., transmission through the Internet).

The present disclosure has been described with reference to embodiments shown in the drawings, but this is only exemplary, and it will be understood by those skilled in the art that various modifications and other equivalent embodiments are possible therefrom. Accordingly, the technical protection scope of the present disclosure should be determined by the technical spirit of the appended claims.

## Claims

1. A method of guiding a use period of a sensor, the method comprising:
determining one or more sensors possessed by a user;
determining whether a remaining usage period of the one or more sensors has reached an order guidance period; and
when the remaining usage period of the one or more sensors has reached the order guidance period, providing an order guidance message to the user,
wherein the one or more sensors are continuously usable for a set use period.

2. The method of guiding the use period of the sensor according to claim 1, wherein the determining of whether the remaining usage period of the one or more sensors possessed by the user has reached the order guidance period comprises:
calculating a total usage period of the one or more sensors possessed by the user based on a usage period of each sensor possessed by the user and a number of the one or more sensors possessed by the user; and
determining whether the remaining usage period of the one or more sensors possessed by the user has reached the order guidance period based on the total usage period.

3. The method of guiding the use period of the sensor according to claim 2, wherein:
the determining of whether the remaining usage period of the one or more sensors has reached the order guidance period further comprises determining whether one of the one or more sensors possessed by the user is used, and
the total usage period of the one or more sensors possessed by the user is calculated based on the usage period of each sensor possessed by the user and the number of the one or more sensors possessed by the user based on a time point when the user uses the one of the one or more sensors.

4. The method of guiding the use period of the sensor according to claim 3, further comprising:
additionally registering a new sensor during use of the one or more sensors;
when the new sensor is additionally registered, changing information of the one or more sensors possessed by the user; and
re-calculating the total usage period of the one or more sensors possessed by the user based on the usage period of each sensor possessed by the user and the number of the one or more sensors possessed by the user,
wherein the determining of whether the remaining usage period of the one or more sensors has reached the order guidance period is performed based on the re-calculated total usage period of the one or more sensors.

5. The method of guiding the use period of the sensor according to claim 2, further comprising:
determining whether a sensor registered as a sensor currently being used by the user is removed;
at the time of removing the sensor registered as the sensor currently being used by the user, determining whether a remaining usage period of the sensor registered as the sensor currently being used by the user exists; and
when the remaining usage period of the sensor currently being used exists, re-calculating the total usage period of the one or more sensors by subtracting the remaining usage period of the sensor currently being used from the total usage period of the one or more sensors possessed by the user,
whether the remaining usage period of the one or more sensors has reached the order guidance period is determined based on the re-calculated total usage period of the one or more sensors.

6. The method of guiding the use period of the sensor according to claim 5, wherein the determining of whether the sensor registered as the sensor currently being used by the user is removed comprises, when a sensor different from the sensor registered as the sensor currently being used is newly registered to be used, determining that the sensor registered as the sensor currently being used is removed.

7. The method of guiding the use period of the sensor according to claim 1, wherein the determining of whether the remaining usage period of the one or more sensors has reached the order guidance period comprises:
determining whether a last sensor is being currently used among the one or more sensors possessed by the user based on use history of the one or more sensors possessed by the user;
when it is determined that the last sensor is being currently used among the one or more sensors possessed by the user, determining a remaining usage period of the last sensor; and
determining whether the remaining usage period of the last sensor has reached the order guidance period.

8. The method of guiding the use period of the sensor according to claim 7, further comprising:
additionally registering a new sensor during use of the one or more sensors; and
when the new sensor is additionally registered, changing information of the one or more sensors possessed by the user,
wherein whether the last sensor is being currently used among the one or more sensors possessed by the user is determined based on the use history of the one or more sensors possessed by the user.

9. The method of guiding the use period of the sensor according to any one of claims 1 to 8, wherein the one or more sensors are one or more sensors for continuous measurement of blood glucose.

10. The method of guiding the use period of the sensor according to any one of claims 1 to 8, wherein:
the one or more sensors are provided to be packaged in a box,
a code corresponding to each of the packaged one or more sensors is disposed on or in the box,
a sensor identifier of each of the one or more sensors is acquired from the code, and
the packaged one or more sensors are registered as the one or more sensors possessed by the user based on the acquired sensor identifier.

11. The method of guiding the use period of the sensor according to any one of claims 1 to 8, wherein:
a code having a sensor identifier is disposed on each of one or more sensors,
the sensor identifier is acquired from the code,
the one or more sensors are registered as the one or more sensors possessed by the user based on the acquired sensor identifier.

12. The method of guiding the use period of the sensor according to any one of claims 1 to 8, further comprising:
providing a list of the one or more sensors possessed by the user to the user in response to a request of the user; and
when a deletion request for selecting a sensor which the user requests to delete from the list is input, updating the list by deleting the selected sensor from the list,
whether the remaining usage period of the one or more sensors possessed by the user has reached the order guidance period is determined based on the updated list.
